# EUROPEAN PATENT APPLICATION

(11) **EP 4 075 136 A1**
(43) Date of publication of application: **19.10.2022**
(21) Application number: 21889585.2
(22) Date of filing: 04.11.2021
(51) Int. Cl.: G01N 33/558, G01N 33/543, G01N 33/68

(54) **RAPID DETECTION KIT FOR ANTI-TNF-ALPHA DRUG MONITORING**

(30) Priority: 09.11.2020 KR 20200148652
(71) Applicant: Boditech Med Inc., Chuncheon-si, Gangwon-do 24398 (KR)
(72) Inventor: CHOI, Eui Yul, Chuncheon-si, Gangwon-do 24214 (KR); LEE, Yoon Suk, Chuncheon-si Gangwon-do 24414 (KR); LEE, Mi Sook, Chuncheon-si Gangwon-do 24239 (KR); CHON, Hyang Ah, Chuncheon-si Gangwon-do 24379 (KR); SAYYED, Danishmalik Rafiq, Chuncheon-si Gangwon-do 24311 (KR)
(74) Representative: De Vries & Metman
(86) International application number: PCT/KR2021/015905
(87) International publication number: WO 2022/098120

(57) **Abstract**

The present invention provides a rapid detection kit for monitoring anti-TNF-α drugs including a porous membrane; a sample pad disposed at the upstream end of the porous membrane and absorbing a sample to provide the sample to the porous membrane; a test line formed on the porous membrane to be spaced apart from the sample pad; and an absorption pad disposed at the downstream end of the porous membrane and absorbing the sample provided from the sample pad, wherein in the test line, a target material including the anti-TNF-α drug is detected, and the target material including the anti-TNF-α drug is a complex including the anti-TNF-α drug and an anti-idiotype antibody against the anti-TNF-α drug. According to the present invention, it is possible to provide an optimized treating method for TNF-α related diseases to patients by providing a kit capable of rapidly detecting anti-TNF-α drugs and accurately measuring the concentration of anti-TNF-α drugs in a sample.

## Description

### [Technical Field]

The present invention relates to a rapid detection kit and more particularly, to a rapid detection kit capable of detecting and measuring the presence or level of anti-tumor necrosis factor (TNF)-α drugs in a sample such as blood and the like.

### [Background Art]

For treatment of inflammatory bowel disease (IBD) or rheumatoid arthritis (RA), as antibodies against a tumor necrosis factor (TNF)-α which is an inflammatory mediator causing IBD or RA, etanercept (Enbrel PFS), infliximab (Remicade), adalimumab (Humira), golimumab (Simponi PFS), and the like have been developed.

However, these anti-TNF-α drugs are very expensive and may cause infections such as tuberculosis. Also, it is known that in about one-third of patients, there is no therapeutic effect by anti-TNF-a drugs, and the therapeutic effect decreases even if treatment is continued in patients who have a therapeutic effect.

As the main cause of the decrease in the therapeutic effect, a decrease in the concentration of the anti-TNF-α drugs in blood, the formation of human antibodies against the anti-TNF-α drugs in blood, etc. are known. Therefore, in order to increase the therapeutic effect, it is necessary to measure the concentration of the anti-TNF-a drugs in blood through therapeutic drug monitoring (TDM) and provide appropriate feedback to the patient.

In particular, enzyme-linked immunosorbent assay (ELISA) is widely used as a test method for the presence and concentration of the anti-TNF-α drugs in blood. However, this method requires a complicated process of reacting various reagents in an exact volume for a specific time in a 96-well plate. In addition, expensive equipment must be used in the laboratory of large hospital or professional personnel must perform the examination. In addition, since the test takes a long time, it takes more than two weeks for the test result to be reflected until the actual treatment. Therefore, the patient has the inconvenience of having to visit the hospital at least twice for examination and treatment, and there is a problem in that the patient is not provided with an accurate treatment method due to the time difference between the examination and treatment.

Therefore, for appropriate treatment, a point-of-care test (POCT) capable of rapid therapeutic drug monitoring is required. A POCT of infliximab is limited to two products (Quantum Blue^{®} Infliximab and RIDA^{®}IFX Monitoring) worldwide, and there are no products that can be used for whole blood. In addition, these products require additional procedures for reacting the sample with the test reagent until the concentration in the blood is measured, so their usefulness as a point-of-care test is limited.

### [Technical Problem]

Accordingly, an object of the present invention is to provide a kit capable of rapidly detecting an anti-TNF-α drug.

Another object of the present invention is to provide a kit capable of accurately measuring the concentration of an anti-TNF-α drug in a sample by minimizing TNF interference.

### [Technical Solution]

An aspect of the present invention provides a rapid detection kit for monitoring an anti-TNF-α drug comprising a porous membrane; a sample pad disposed at the upstream end of the porous membrane and absorbing a sample to provide the sample to the porous membrane; a test line formed on the porous membrane to be spaced apart from the sample pad; and an absorption pad disposed at the downstream end of the porous membrane and absorbing the sample provided from the sample pad, wherein in the test line, a target material including the anti-TNF-α drug is detected, and the target material including the anti-TNF-α drug is a complex having the anti-TNF-α drug and an anti-idiotype antibody against the anti-TNF-α drug.

At the test line, a linker or an anti-idiotype antibody against the anti-TNF-α drug may be immobilized.

The linker may be at least one selected from the group consisting of streptavidin, avidin, biotin, a protein containing a histidine functional group, and an anti-histidine antibody.

The rapid detection kit may further include a fluorescence detector capable of detecting fluorescence generated in the test line or a control line.

The sample may include an antibody-drug complex consisting of an anti-TNF-α drug and an anti-human IgG antibody labeled with a fluorescent moiety.

The sample may include an antibody-drug complex consisting of an anti-TNF-α drug and an anti-idiotype antibody against the anti-TNF-α drug labeled with a fluorescent moiety.

The sample may include an antibody-drug-antibody complex consisting of an anti-TNF-α drug, an anti-idiotype antibody against the anti-TNF-α drug labeled with a fluorescent moiety, and a tracer antibody.

### [Advantageous Effects]

According to the present invention, it is possible to provide an optimized treating method for TNF-α related diseases to patients by providing the kit capable of rapidly detecting an anti-TNF-α drug and accurately measuring the concentration of the anti-TNF-α drug in a sample without TNF-α interference.

### [Description of Drawings]

FIG. 1A is a schematic diagram illustrating the binding of a drug and a target to which the drug acts, and FIG. 1B is a schematic diagram illustrating that the anti-idiotypic antibody binds to a site different from the site where the target binds to the drug.
FIG. 2A illustrates a porous membrane on which a sample pad, a control line, a test line, and an absorption pad are formed, and FIG. 2B illustrates an example of a plastic housing covering the porous membrane.
FIG. 3 is a schematic diagram illustrating how to use the rapid detection kit according to an embodiment of the present invention.
FIG. 4 is a schematic diagram illustrating examples in which target materials are immobilized at the test line in the present invention.
FIG. 5A is a schematic diagram illustrating that signals are detected from the test line and the control line, and FIG. 5B is a schematic diagram illustrating that the intensity of the signal to be detected from the test line increases as the drug concentration increases.
FIG. 6 shows the analysis of the change in the IFX detection value according to the TNF-α concentration in the evaluation sample based on the IFX detection value of the sample in which TNF-α does not exist.

### [Best Mode for the Invention]

A rapid drug detection kit of the present invention uses lateral flow assays (LFA) to measure the presence and concentration of an anti-TNF-α drug in a sample. The rapid drug detection kit comprises a porous membrane, a sample pad, a test line, a control line, an absorption pad, and a fluorescence detector.

The anti-TNF-α drug is an agent that suppresses or inhibits TNF-α activity, and may be, for example, etanercept (Enbrel PFS), infliximab (Remicade), adalimumab (Humira), and golimumab (Simponi PFS).

The sample may include blood, blood cells, lymph, saliva, urine, sweat, interstitial or intracellular body fluid, or materials extracted therefrom. The blood may include blood, plasma, or serum that has been subjected to a predetermined treatment (e.g., coagulation prevention), but is not limited thereto. The sample may be used in a manipulated or non-manipulated state.

The detection buffer is a buffer containing a test detector antibody, a control detector antibody, or a tracer antibody. The detection buffer is mixed with the sample and provided to the detection kit.

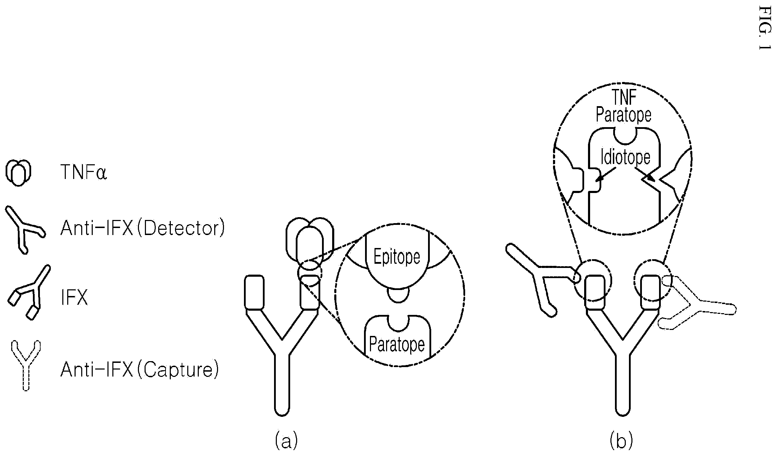

The test detector antibody is an anti-idiotype antibody against the anti-TNF-α drug labeled with a fluorescent moiety or an anti-human IgG antibody labeled with a fluorescent moiety. As illustrated in FIG. 1, the anti-idiotype antibody refers to an antibody that binds to a site other than the binding site of the target on which the drug acts. By using the anti-idiotype antibody, the concentration of the anti-TNF-α drug in the sample may be accurately measured regardless of whether the anti-TNF-α drug binds to TNF-α.

The fluorescent moiety refers to a fluorescent molecule or a derivative thereof that generates a fluorescence signal by absorbing light (e.g., UV light) of a specific frequency. The fluorescent moiety may be Alexa 350, Alexa 405, Alexa 430, Alexa 488, Alexa 555, Alexa 647, AMCA, aminoacridine, BODIPY 630/650, BODIPY 650/665, BODIPY-FL, BODIPY-R6G, BODIPY-TMR, BODIPYTRX, 5-carboxy-4',5'-dichloro-2',7'-dimethoxyfluores-cein, 5-carboxy-2',4',5',7'-tetrachlorofluorescein, 5-carbox-yfluorescein, 5-carboxyrhodamine, 6-carboxyrhodamine, 6-car-boxytetramethylrhodamine, cascade blue, Cy2, Cy3, Cy5, Cy7, 6-FAM, dansyl chloride, fluorescein, HEX, 6-JOE, NBD (7-nitro-benzo-2-oxa-1,3-diazole), Oregon Green 488, Oregon Green 500, Oregon Green 514, Pacific Blue, phthalic acid, terephthalic acid, isophthalic acid, cresyl fast violet, cresyl violet, brilliant cresyl blue, 4-aminobenzoic acid, erythrosine, phthalocyanine, azomethine, cyanine, xanthine, succinyl fluorescein, rare earth metal cryptate, tri-bipyridyldiamine Europium, Europium cryptate compound or chelate, diamine, dicyanine, La Jolla blue dye, allophycocyanin, allococyanin B, phycocyanin C, phycocyanin R, thiamine, R-pycoerythrin, C-phycocyanin, phycoerythrin R, REG, Rhodamine Green, Rhodamine isothiocyanate, Rhodamine Red, ROX, TAMRA, TET, TRIT (tetramethylrhodamine isothiol), tetramethylrhodamine, Texas Red, CdS/ZnS Quantum dot, CdSe Quantum dot, CdSe/ZnS Quantum dot, CdSSe/ZnS Quantum dot, CdTe/CdSe/ZnS Quantum dot, CdS Quantum dot, ZnCdSe/ZnS Quantum dot, CdTe Quantum dot, Eu and Tb.

The control detector antibody may be chiken IgY, antichiken IgY, KLH, anti-KLH, rabbit IgG, anti-rabbit IgG, goat IgG, and anti-goat IgG which are labeled with the fluorescent moiety. The fluorescent moiety included in the control detector antibody may be the same as or different from the fluorescent moiety included in the test detector antibody.

The tracer antibody is an anti-idiotype antibody to which a linker binds. The linker may be any one or more selected from the group consisting of biotin, streptavidin, neutravidin, avidin, protein A/G, glutathione, a protein containing a histidine functional group, and an anti-histidine antibody.

The porous membrane is a membrane capable of providing capillary motion or lateral flow. The porous membrane may be nitrocellulose blends (e.g., nitrocellulose, polyester or cellulose), porous paper, rayon, glass fiber, acrylonitrile copolymer, nylon, and the like.

The sample pad is disposed at the upstream end of the porous membrane. The sample pad absorbs the sample mixed with the detection buffer and causes it to flow along the porous membrane.

The test line is formed on the porous membrane and spaced apart from the sample pad. At the test line, a linker or an anti-idiotype antibody against the anti-TNF-α drug is immobilized as a test capture. In the test line, a target material including the anti-TNF-α drug is detected. The target material is an antibody-drug complex or an antibody-drug-antibody complex. More specifically, the target material may be an (anti-idiotype antibody)-(anti-TNF-α drug) complex, an (anti-human IgG antibody)-(anti-TNF-α drug) complex, or an (anti-idiotype antibody)-(anti-TNF-α drug)-(tracer antibody) complex.

The control line is formed on the porous membrane and spaced apart from the sample pad. The control line may be immobilized with a control capture, such as chiken IgY, antichiken IgY, KLH, anti-KLH, rabbit IgG, anti-rabbit IgG, goat IgG, anti-goat IgG, and the like. The control line detects the control detector present in the sample.

The absorption pad is disposed at the downstream end of the porous membrane and absorbs the sample provided from the sample pad.

The fluorescence detector detects fluorescence generated from the fluorescent moiety. For example, the fluorescence detector is a photodetector capable of measuring the intensity of a fluorescence signal by irradiating excitation light to the fluorescent moieties of the test line and the control line through a separate path to excite the fluorescent moieties and detecting the emitted fluorescence.

Hereinafter, embodiments of the present invention will be described in detail with reference to the accompanying drawings. However, the following embodiments are just illustrative of the present invention, and the scope of the present invention is not limited to the following embodiments.

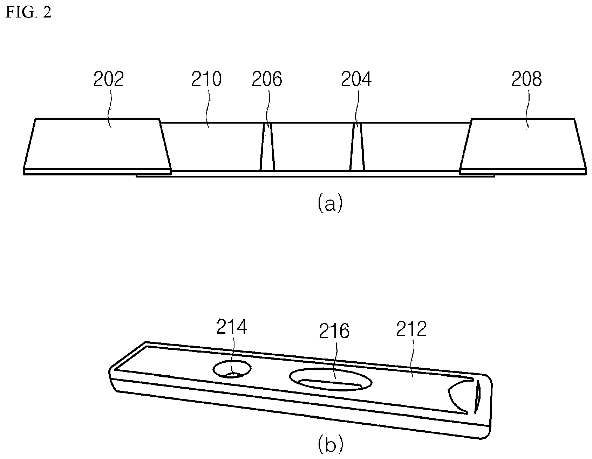

FIG. 2A illustrates a porous membrane 210 on which a sample pad 202, a control line 204, a test line 206, and an absorption pad 208 are formed, and FIG. 2B illustrates an example of a plastic housing 212 covering the porous membrane 210. The plastic housing 212 includes a sample inlet 214 and a measurement window 216.

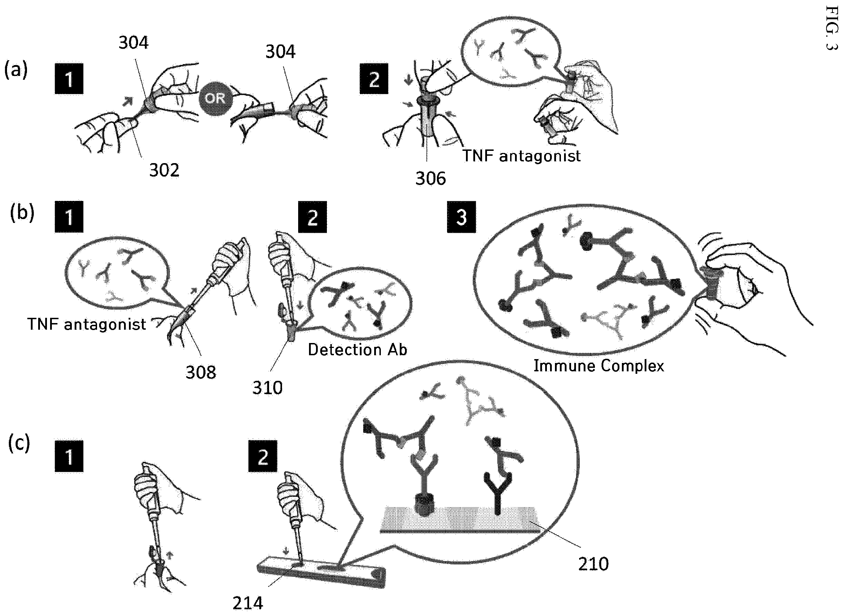

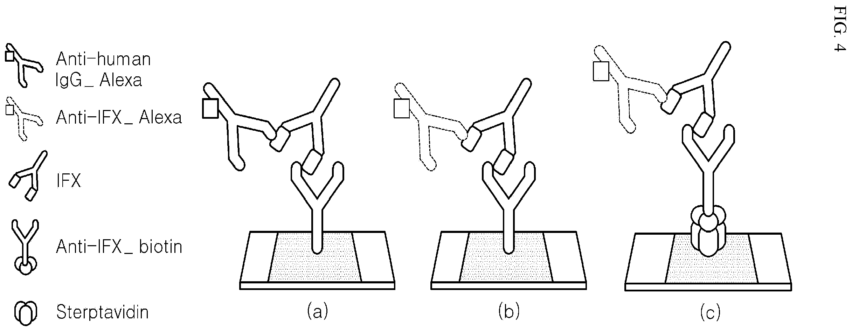

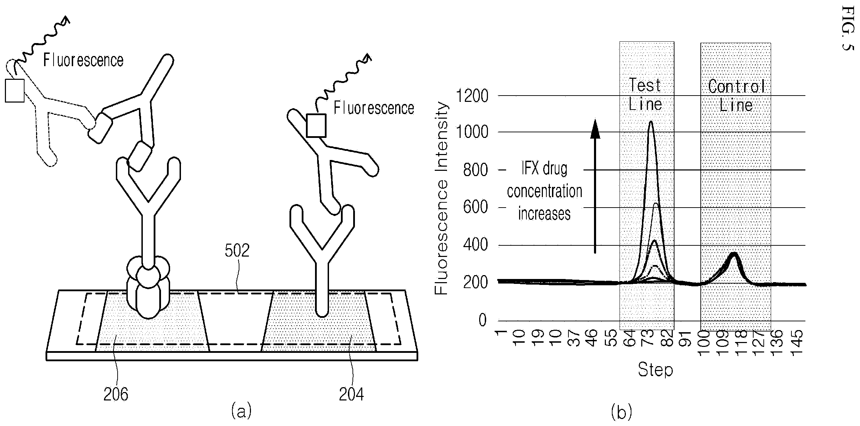

FIG. 3 is a schematic illustrating a method of using the rapid detection kit according to an embodiment of the present invention, and FIG. 4 is a schematic diagram illustrating examples in which a target material is immobilized at the test line 206. FIG. 5A is a schematic diagram illustrating that signals are detected from the test line 206 and the control line 204, and FIG. 5B is a schematic diagram illustrating that the intensity of the signal to be detected from the test line increases as the drug concentration increases.

### 1-1. First Embodiment

In the first embodiment, an anti-idiotype antibody against an anti-TNF-α drug is immobilized on the test line 206, and an anti-human IgG antibody labeled with a fluorescent moiety as a test detector antibody is included in the detection buffer. The anti-human IgG antibody labeled with the fluorescent moiety reacts with the anti-TNF-α drug in the sample to form an antibody-drug complex, and the antibody-drug complex reacts with the anti-idiotype antibody against the anti-TNF-α drug immobilized at the test line 206 and binds to the anti-idiotype antibody while flowing along the porous membrane 210. The schematic diagram of the first embodiment is illustrated in FIG. 4A.

### 1-2. Second Embodiment

In the second embodiment, an anti-idiotype antibody against an anti-TNF-α drug is immobilized at the test line 206, and the anti-idiotype antibody against the anti-TNF-α drug labeled with a fluorescent moiety as a test detector antibody is included in the detection buffer. The anti-idiotype antibody against the anti-TNF-α drug labeled with the fluorescent moiety reacts with the anti-TNF-α drug in the sample to form an antibody-drug complex, and the antibody-drug complex reacts with the anti-idiotype antibody against the anti-TNF-α drug immobilized at the test line 206 and binds to the anti-idiotype antibody while flowing along the porous membrane 210. The schematic diagram of the first embodiment is illustrated in FIG. 4B.

### 1-3. Third Embodiment

In the third embodiment, streptavidin is immobilized at the test line 206, and an anti-idiotype antibody against an anti-TNF-α drug labeled with a fluorescent moiety as a test detector antibody and an anti-idiotype antibody binding to biotin as a tracer antibody are included in the detection buffer. The anti-TNF-α drug in the sample reacts with the anti-idiotype antibody against the anti-TNF-α drug labeled with the fluorescent moiety and the tracer antibody to form an antibody-drug-antibody complex, and the antibody-drug-antibody complex reacts with streptavidin immobilized at the test line 206 and binds to the streptavidin while flowing along the porous membrane 210. The schematic diagram of the third embodiment is illustrated in FIG. 4C.

### 2. Selection of anti-idiotype antibody

In the present embodiment, the process of selecting the anti-idiotype antibody is as follows.

First, a total of 16 monoclonal anti-IFX (anti-infliximab) antibodies binding to infliximab were developed. In addition, competitive ELISA was performed to identify whether these antibodies against infliximab share the binding site of TNF-a with infliximab or not.

The principle of competitive ELISA is to evaluate the reactivity of anti-IFX against infliximab binding to TNF-α. First, 0.5 µg/mL of infliximab is coated

to each well of a 96-well plate, and left at 4°C for 12 hours. The 96-well plate in which infliximab is immobilized is washed with 0.1% phosphate-buffered saline with Tween 20 (PBST), and is blocked with a constant concentration of a bovine serum albumin (BSA) (in 0.1% PBST) solution for 1 hour at room temperature. After blocking, the 96-well plate is washed with 0.1% PBST, and a reaction with 1 µg/mL TNF-α proceeds. At this time, TNF-α reacts with the IFX immobilized on the bottom of the 96-well plate. After the reaction ends, the 96-well plate is washed with 0.1% PBST, and a reaction with each of 16 monoclonal anti-IFX antibody solutions at 1 µg/mL is carried out. When the reaction of the monoclonal anti-IFX antibodies and IFX ends, the 96-well plate is washed with 0.1% PBST, and a reaction with a secondary antibody labeled with horseradish peroxidase (HRP) is performed for color development. After the reaction is completed, the 96-well plate is washed with 0.1% PBST. Next, color development is performed using TMB (tetramethylbenzidine), and then absorbance is measured after color development is completed with a stop solution.

The measurement results of the 16 monoclonal anti-IFX antibodies for identifying antibody binding sites are shown in Table 1 below.

**[Table 1]**

| Clone No. | O.D. | | Recovery |
|---|---|---|---|
| | IFX (Control) | IFX+TNF | |
| 5E36 | 1.97 | 1.39 | 71% |
| 6E60 | 2.6 | 2.33 | 90% |
| 2A86 | 2.1 | 2.19 | 104% |
| 4C18 | 1.82 | 1.32 | 73% |
| 4G36 | 2.31 | 2.34 | 101 % |
| 1-11B46 | 2.13 | 1.93 | 91% |
| 4H78 | 2.46 | 2.4 | 98% |
| 10D2 | 1.52 | 0.63 | 41% |
| 2C44 | 2.75 | 2.78 | 101% |
| 7A47 | 2.71 | 2.59 | 96% |
| 3B46 | 2.71 | 2.35 | 87% |
| 5B63 | 2.48 | 2.51 | 101% |
| 2F5 | 2.93 | 2.74 | 94% |
| 4H41 | 3.04 | 2.78 | 91 % |
| 7E60 | 2.66 | 2.57 | 97% |
| 8G73 | 2.72 | 2.16 | 79% |

As a result of identifying the binding site of each monoclonal antibody, 4 clones (5E36, 4C18, 10D2, and 8G73) of a total of 16 clones exhibited a 20% or more decrease in reactivity to IFX upon binding of IFX and TNF-α. It is presumed that since they share some or all of the binding sites with TNF-α, their reactivity is reduced by interference with TNF-α. Therefore, the remaining 12 types were determined to be anti-idiotype antibodies that do not share the binding sites of IFX and TNF-α.

Thereafter, the final anti-idiotype antibody was selected through the IFX reactivity evaluation and antibody pair selection process among these 12 types of anti-idiotypic antibodies.

### 3-1. Preparation of anti-idiotype antibody labeled with fluorescent moiety

An anti-idiotype antibody labeled with a fluorescent moiety is prepared. An appropriate amount of NaHCO₃ is dissolved in the prepared anti-idiotype antibody, and an appropriate amount of fluorescent moiety-NHS (N-hydroxysuccinimide) ester (e.g., alexa-NHS ester) is added and reacted. From the reacted solution, only the anti-idiotype antibody labeled with the fluorescent moiety is separated using size exclusion chromatography.

### 3-2. Preparation of tracer antibody

A tracer antibody, which is an anti-idiotype antibody to which a linker binds, is prepared. An appropriate amount of NaHCO₃ is dissolved in the anti-idiotype antibody, and an appropriate amount of biotin-NHS is added and reacted. From the reacted solution, only the biotin-TNF-α complex is separated using a centrifuge.

### 4. Preparation of rapid drug detection kit

The rapid drug detection kit of the present embodiment comprises a porous membrane 210, a sample pad 202, a test line 206, a control line 204, an absorption pad 208, and a fluorescence detector (not illustrated). The control line 204 and the test line 206 are formed on the porous membrane 210 by dispensing and drying a control capture and a test capture at a predetermined concentration. The control capture is chicken IgY or KLH, and the test capture is an anti-idiotype antibody or linker. A suitable sample pad 202 is disposed at the upstream end of the porous membrane 210, and a suitable absorption pad 208 is disposed at the downstream end of the porous membrane 210. Thereafter, the porous membrane 210 on which the sample pad 202 and the absorption pad 208 are formed is put into a plastic housing 212 and assembled.

### 5. Using method of rapid anti-TNF-α drug detection kit

The method of using the rapid detection kit according to an embodiment of the present invention comprises three steps as shown in FIG. 3.

FIG. 3A shows the steps of diluting a sample. As a sample 302, capillary blood, serum, plasma, and whole blood containing an anti-TNF-α drug collected from a finger or the like may be used. 10 µl of the sample is collected using a sample collector 304 and mixed with a diluent 306.

FIG. 3B shows the steps of preparing a sample. The sample dilution solution 308 prepared in FIG. 3A is added to a detection buffer 310. At this time, the detection buffer 310 includes a test detector antibody, a control detector, a tracer antibody, and the like to form an antibody-drug complex or an antibody-drug-antibody complex, that is, a target material including an anti-TNF-α drug.

FIG. 3C shows the steps of immobilizing the formed complex to the porous membrane 210. The sample prepared in FIG. 3B is put into the sample inlet 214 of the plastic housing 212. The sample flows along the porous membrane 210. The control detector is immobilized at the control line 204, and the target material including the anti-TNF-α drug is immobilized at the test line 206. More specifically, since the drug of the antibody-drug complex binds to the anti-idiotype antibody immobilized at the test line 206, the target material is immobilized at the test line 206. In addition, since the linker of the antibody-drug-antibody complex binds to the linker immobilized at the test line 206, the target material is immobilized at the test line 206.

### 6-1. Fluorescence signal processing and result analysis

After a certain incubation time has elapsed and the immobilization reaction of the target material and the control detector on the porous membrane 210 is finished, as shown in Figure 5(a), the fluorescence signal generated from the test line 206 and the control line 204 is scanned by a fluorescence detection unit (not shown). The test line 206 and the control line 204 are positioned in a fluorescence signal measurement area 502, and the fluorescence signal measurement area 502 is positioned in the measurement window 216. As shown in FIG. 5B, as the concentration of the drug in the sample increases, the amount of the target material immobilized at the test line 206 increases, so that the fluorescence signal also increases. On the other hand, the control line 204 shows a constant fluorescence signal regardless of the concentration of the drug in the sample.

The concentration of the drug is calculated by analyzing the concentration of an unknown sample based on the fluorescence signal of a standard sample. This method is different from the conventional ELISA test method in which a test sample is simultaneously tested with standard and quality control materials, and it is economical because there is no consumption of additional resources other than reagents for testing the sample to be verified.

### 6-2. Quantitative analysis of anti-TNF-α drug using rapid detection kit

A quantitative analysis result of the rapid detection kit for monitoring the anti-TNF-α drug is derived through the following process.

First, 5 to 6 standard samples or calibrators diluted at a predetermined concentration using an infliximab international standard are prepared. The most important purpose of the drug monitor is to maintain blood circulation drug within a maximum concentration (therapeutic range) without side effects while having the effects and a minimum concentration (trough level) to be maintained before the next injection. Therefore, the concentration of the calibrator is set to include a concentration lower than the trough level, the trough level, and low/middle/high regions based on the therapeutic range. In the embodiment, IFX calibrators of 0, 0.5, 2, 20, and 50 µg/mL concentrations were prepared.

Next, the concentration of the sample prepared according to 5. using method of rapid anti-TNF-α drug detection kit described above is measured with the rapid detection kit. When the concentration of the drug in the sample increases, the fluorescence signal of the test line 206 increases, whereas the control line 204 is not affected by the drug concentration to exhibit a constant fluorescence signal regardless of the concentration. A calibration curve is prepared by calculating a ratio of the fluorescence signals of the test line 206 and the control line 204 measured by the fluorescence detector.

The prepared calibration curve is used to determine the concentration of the unknown sample. Specifically, the concentration of the unknown sample is calculated by estimating the result value of the fluorescence signal ratio obtained from the unknown sample to the calibration curve.

### 7. Interference exclusion performance of TNF-α of rapid detection kit

The rapid detection kit of the present embodiment uses an anti-idiotype antibody to exclude the interference of TNF-α. An appropriate anti-idiotypic antibody was selected through the experiment, and the TNF-α interference exclusion performance of the finally prepared rapid detection kit was confirmed.

Inflammatory bowel disease (IBD) patients have a very high level of TNF-α in the blood than healthy people, and it is known that the level is several to several tens of pg/mL. Therefore, in order to evaluate the TNF-α interference exclusion performance of the rapid detection kit of the present embodiment, an experiment was conducted to determine whether there was TNF-α interference in the presence of TNF-α above ng/mL level.

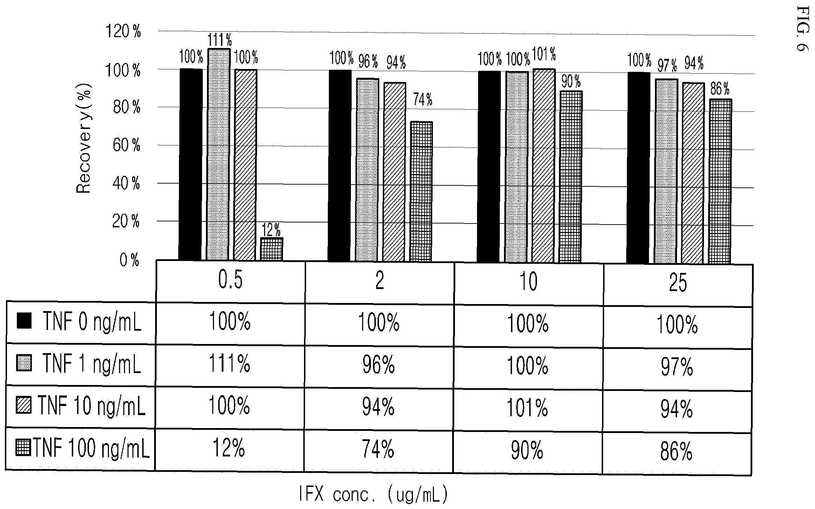

IFX (infliximab) solutions of four concentrations (0.5, 2, 10, and 25 µg/mL) were prepared and 0, 1, 10, and 100 ng/mL of TNF-α solutions were added to each solution to prepare evaluation samples. At this time, the volume of the added TNF-α solution was less than 10% of the total volume of the evaluation samples. Based on the IFX detection result of the sample in which TNF-α does not exist (TNF-α 0 ng/mL), the analysis result of the change (recovery) in the IFX detection value according to the TNF-α concentration in the evaluation sample is shown in Figure 6.

As shown in FIG. 6, in the rapid detection kit of the present embodiment, there was almost no interference by TNF-α in the sample. However, it was confirmed that the interference phenomenon appears only in some cases where TNF-α is present at a very high concentration (100 ng/mL) and the IFX concentration is at a normal trough level or therebelow (0.5 to 2 (µg/mL). On the other hand, it was confirmed that even if TNF-α exists at a very high concentration (100 ng/mL), the interference phenomenon is alleviated at a high concentration of IFX 10 µg/mL or more.

While the present invention has been particularly illustrated and described with reference to preferred embodiments thereof, it will be understood by those skilled in the art that various changes in forms and details may be made without departing from the scope of the present invention included in the appended claims. Also, it should be understood that the embodiments described herein are not mutually exclusive, and features of various embodiments may be combined in whole or in part according to the present invention.

## Claims

1. A rapid detection kit for monitoring an anti-TNF-α drug comprising:
a porous membrane;
a sample pad disposed at the upstream end of the porous membrane and absorbing a sample to provide the sample to the porous membrane;
a test line formed on the porous membrane to be spaced apart from the sample pad; and
an absorption pad disposed at the downstream end of the porous membrane and absorbing the sample provided from the sample pad,
wherein in the test line, a target material including the anti-TNF-α drug is detected, and
the target material including the anti-TNF-α drug is a complex including the anti-TNF-α drug and an anti-idiotype antibody against the anti-TNF-α drug.

2. The rapid detection kit of claim 1, wherein at the test line, a linker or an anti-idiotype antibody against the anti-TNF-α drug is immobilized.

3. The rapid detection kit of claim 2, wherein the linker is at least one selected from the group consisting of streptavidin, avidin, biotin, a protein containing a histidine functional group, and an anti-histidine antibody.

4. The rapid detection kit of claim 3, further comprising:
a fluorescence detector capable of detecting fluorescence generated at the test line or the control line.

5. The rapid detection kit of any one of claims 1 to 4, wherein the sample includes an antibody-drug complex consisting of an anti-TNF-α drug and an anti-human IgG antibody labeled with a fluorescent moiety.

6. The rapid detection kit of any one of claims 1 to 4, wherein the sample includes an antibody-drug complex consisting of an anti-TNF-α drug and an anti-idiotype antibody against the anti-TNF-α drug labeled with a fluorescent moiety.

7. The rapid detection kit of any one of claims 1 to 4, wherein the sample includes an antibody-drug-antibody complex consisting of an anti-TNF-α drug, an anti-idiotype antibody against the anti-TNF-α drug labeled with a fluorescent moiety, and a tracer antibody.
